# EUROPEAN PATENT APPLICATION

(11) **EP 1 719 529 A1**
(43) Date of publication of application: **08.11.2006**
(21) Application number: 05703547.9
(22) Date of filing: 13.01.2005
(51) Int. Cl.: A61K 49/00, A61K 51/04, C07D 207/04, C07D 295/18, C07C 251/24, C07C 317/50

(54) **CONTRAST MEDIUM FOR THROMBUS FORMATION**

(30) Priority: 25.02.2004 JP 2004049996
(71) Applicant: Astellas Pharma Inc., Tokyo 103-8411 (JP)
(72) Inventor: MURAKAMI, Yoshihiro c/o Astellas Pharma Inc., Chuo-ku Tokyo 103-8411 (JP); AOKI, Toshiaki c/o Astellas Pharma Inc., Chuo-ku Tokyo 103-8411 (JP); TAKAMATSU, Hiroyuki, Hamamatsu-shi, Shizuoka 4328018 (JP); NISHIMURA, Shintaro c/o Astellas Pharma Inc., Chuo-ku Tokyo 103-8411 (JP); OSODA, Kazuhiko c/o Astellas Pharma Inc., Chuo-ku Tokyo 103-8411 (JP)
(74) Representative: Gille Hrabal Struck Neidlein Prop Roos
(86) International application number: PCT/JP2005/000308
(87) International publication number: WO 2005/079863

(57) **Abstract**

It is intended to provide a contrast medium for thrombus capable of binding specifically to a thrombus, lowering background noise and thus improving the resolution in a contrast examination for thrombus formation; and a method of detecting a thrombus by using the same. A contrast medium for thrombus comprising, as the active substance, a substance obtained by labeling a compound capable of binding to GPIIb/IIIa which is selected from among compounds represented by the general formulae (I) to (IV) and physiologically acceptable salts thereof, and a method of detecting a thrombus by using the same.

## Description

### Technical Field

The present invention relates to a contrast medium for thrombus comprising a compound capable of binding to glycoprotein (GP) IIb/IIIa.

### Background Art

Pathological thrombus formation in blood vessels is a cause of onset of ischemic diseases such as myocardinal infarct, cerebral infarct, peripheral nerve circulation disorder and the like. However, change in thrombus formation with lapse of time and distribution of thrombus in pathology is little known because an imaging method which makes it possible to quantitatively and effectively detect thrombus formation has not yet been established until today. If such an imaging method is established, this method will be useful for an evaluation of beneficial effect of a drug for cerebral infarct or for an investigation of new pathologies for which an effect of antithrombotic drugs or the like is expected.

With regard to the studies for thrombus imaging, there are reports of thrombus imaging in a patient with deep venous thrombosis using a radioactive technetium (^{99m}Tc)-labeled peptide P280 (Muto P. et al., J. Nucl Med. 1995, 36, p.1384-1391), of thrombus imaging in canine vein with a ^{99m}Tc-labeled activated platelet receptor-binding peptide (Lister-James L. et al., J. Nucl Med. 1996, 37, p.775-781), and of deep thrombus imaging in canine vein using radioactive iodine (¹²⁵I)-labeled protein (Knight L.C. et al., Thromb Haemost., 1998, 80, p.845-851).
Thus, it is known that the thrombus imaging can be carried out by using a labeled peptide which shows IC₅₀=0.087 µM or IC₅₀=0.079 µM±0.017 µM in a test of inhibition of adenosine diphosphate (ADP)-induced platelet aggregation (Muto P. et al. and Lister-James L. et al., mentioned above). It is also known that the thrombus imaging can be carried out by using a labeled peptide which shows the affinity to ADP-stimulated platelets (Knight L.C. et al., above).

The main component of a thrombus is platelets, GPIIb/IIIa existing on the membrane thereof. It is known that GPIIb/IIIa is expressed only in platelets and platelet producing cells, and that a resting GPIIb/IIIa and an active GPIIb/IIIa specifically exist in the blood stream and at the site of thrombus formation, respectively. GPIIb/IIIa functions as a receptor of adhesive protein fibrinogen (precursor of fibrin), of fibronectin, of von Willebrand factor and of vitoronectin, and plays an important role for the thrombus formation.

As fibrinogen receptor antagonists, a compound represented by the general formula (I):

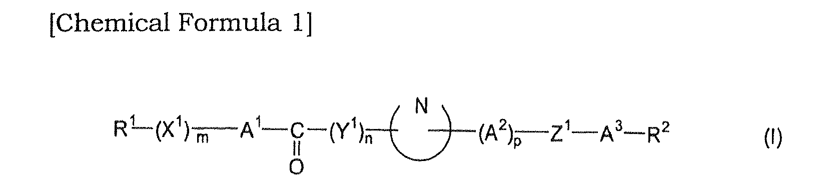

wherein
R¹ represents an N-containing cycloalkyl radical which may have one or more substituents;
R² represents a carboxy or protected carboxy radical;
A¹ represents a lower alkylene, lower alkanyl-ylidene or lower alkenylene radical, each of which may have one or more substituents; A² represents a lower alkylene radical;
A³ represents a lower alkylene radical which may have one or more substituents;
a moiety represented by

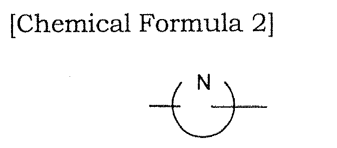

is a N-containing heterocyclic radical represented by the formula:

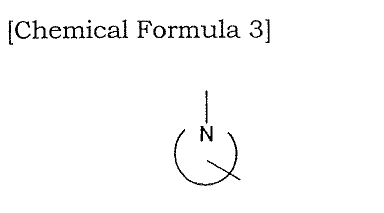

which may have one or more substituents;
X¹ represents O, S or NH;
Y¹ represents NH; and
Z¹ represents

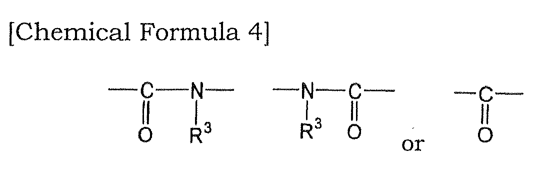

wherein R³ represents a hydrogen atom or a lower alkyl radical; and
m, n and p are the same or different and represent an integer of 0 or 1, respectively;
a compound represented by the general formula (II):

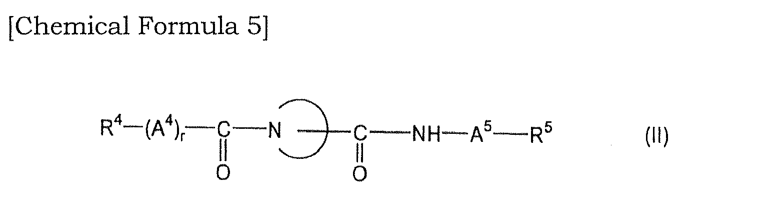

wherein
R⁴ represents a piperidyl, tetrahydropyridyl, azetidinyl or tetrahydroisoquinolyl radical and these piperidyl, tetrahydropyridyl, azetidinyl and tetrahydroisoquinolyl radicals may have an amino protective group;
R⁵ represents a carboxy or protected carboxy radical;
A⁴ represents a lower alkylene, lower alkanyl-ylidene, lower alkenylene, cyclo(lower)alkylene or arylene radical;
A⁵ represents a lower alkylene radical which may have one or more substituents or an arylene radical;
a moiety represented by

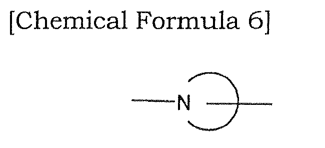

represents a piperidinediyl or tetrahydroisoquinolinediyl radical; and
r represents an integer of 0 or 1; and
a compound represented by the general formula (III):

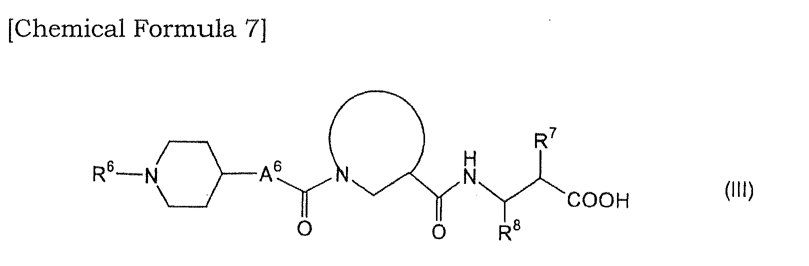

wherein
R⁶ represents a hydrogen atom or an amino protective group;
A⁶ represents a lower alkylene or lower alkenylene radical;
R⁷ represents a hydrogen atom; a lower alkanoyl radical which may be substituted with amino, lower alkanoylamino, ar(lower)alkoxycarbonylamino, aryl, aroylamino, carboxy, lower alkoxycarbonylamino, ar(lower)alkoxy, lower alkoxycarbonyl, lower alkanoyloxy, lower alkoxy or hydroxyl, among which aryl and
aroylamino may further be substituted with carboxy, lower alkoxy or lower alkoxycarbonyl; a lower alkoxycarbonyl radical which may be substituted with lower alkoxy, aryl or cyclo(lower)alkyl; a lower alkenyloxycarbonyl radical; a di(lower)alkylaminosulphonyl radical; a cycloalkanoyl radical which may be substituted with lower alkoxy; an aroyl radical which may be substituted with (C₃-C₆) alkoxy, carbamoyl(lower)alkoxy, N-(lower)alkylcarbamoyl(lower)alkoxy, N,N-di(lower)alkylcarbamoyl(lower)alkoxy, lower alkoxycarbonyl, nitro, cyano, carboxy, carboxy(lower)alkoxy, ar(lower)alkoxy, lower alkoxycarbonyl(lower)alkoxy, cyclo(lower)alkoxy, lower alkoxycarbonylamino, cyclo(lower)alkyl(lower)alkoxy, lower alkanoylamino or lower alkylcarbamoyl; an aryloxycarbonyl radical; a heterocyclylcarbonyl radical; an amino radical which may be substituted with an acyl radical selected from the group consisting of a protected carboxycarbonyl radical and a heterocyclyloxycarbonyl radical;
R⁸ represents a hydrogen atom or an aryl or aralkyl radical which may be substituted with one or more hydroxyl and/or lower alkoxy;
a moiety represented by the formula:

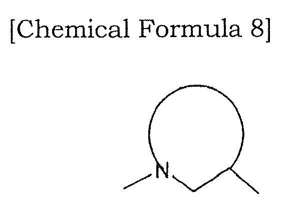

represents a divalent N-containing, 6 to 8-membered heterocyclic radical;
are known (see WO 95/08536, WO96/29309, WO 97/33869 and WO01/60813).
These compounds have been known to be effective for prophylaxis of thrombus formation as a GPIIb/IIIa antagonist, however, the use thereof as a contrast medium for thrombus has not been known.

### Disclosure of the Invention

The objective of the present invention is to provide a contrast medium for thrombus which can specifically bind to the thrombus, decrease a background noise and improve the resolution on thrombus imaging, and a method of thrombus detection using the same.

The present invention is to provide a contrast medium for thrombus which comprises, as an active substance, a substance obtained by labeling a compound capable of binding to GPIIb/IIIa selected from compounds represented by the above general formulae (I) to (III) and the following formula (IV):

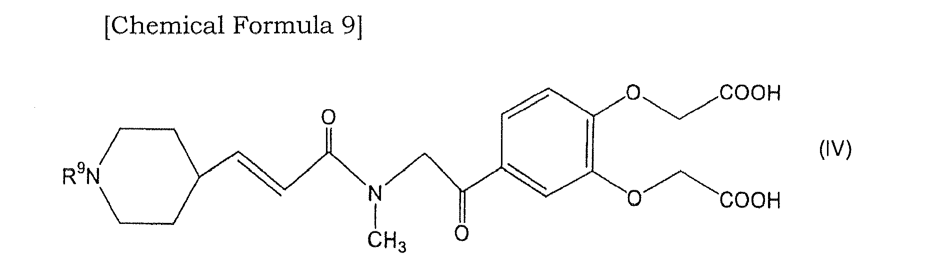

wherein R⁹ represents a hydrogen atom or an amino protective group; and a physiologically acceptable salt thereof.

The present invention also provides a compound represented by the above general formula (IV) and a physiologically acceptable salt thereof.
The present invention further provides a method of detecting a thrombus which comprises the steps of administering the above contrast medium for thrombus to a mammal and detecting a label localized to the thrombus.

### Effect of the Invention

The contrast medium for thrombus of the present invention can specifically bind to a thrombus and thereby has an effect that it makes possible to carry out thrombus imaging with decreased background and improved resolution.

### Best Mode for Carrying Out the Invention

The present invention is a contrast medium for thrombus which comprises, as an active substance, the substance obtained by labeling the compound capable of binding to GPIIb/IIIa.
The above-mentioned compound capable of binding to GPIIb/IIIa may be a compound that has a binding capacity to GPIIb/IIIa which is produced on the surface of platelets, and preferably is a compound which has a binding capacity selectively for an active GPIIb/IIIa. With using such a compound capable of binding to GPIIb/IIIa, it is possible to obtain the contrast medium for thrombus which binds specifically to the active GPIIb/IIIa existing on a membrane of platelets that are main components of the thrombus and which has low binding capacity for a resting GPIIb/IIIa existing in blood stream.

In the present specification, the compound having a binding capacity to GPIIb/IIIa is preferably a compound which can inhibit the aggregation of activated platelets in a method of measuring an inhibitory activity of adenosine diphosphate (ADP)-induced platelet aggregation, described herein below.
The specific binding capacity of the above compound capable of binding to GPIIb/IIIa toward the active GPIIb/IIIa can be determined by calculating an R/A ratio from the measurement result of an inhibitory activity of platelet aggregation mentioned herein below and the measurement result of suppression activity of fibrinogen adhesion of prostaglandin E1 (PGE1)-treated platelet.

The compound capable of binding to GPIIb/IIIa according to the present invention is selected from the compounds represented by the above general formulae (I) to (IV) and physiologically acceptable salts thereof, and preferably is a compound represented by the following formula (III-1):

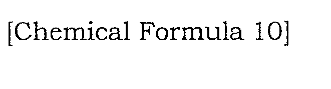

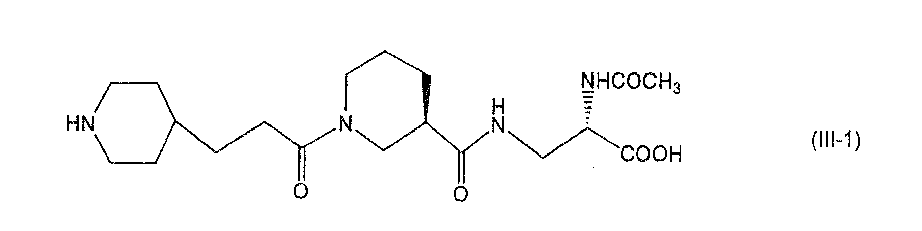

or the compound represented by the above formula (IV) and physiologically acceptable salts thereof.
The compound represented by the above general formula (I) includes the compounds disclosed in WO 95/08536. The compound represented by the above general formula (II) includes the compounds disclosed in WO 96/29309. The compound represented by the above general formula (III) includes the compounds disclosed in WO 97/33869, WO 01/60813 and WO 00/21932.

As the compound capable of binding to GPIIb/IIIa, compounds disclosed in WO 95/25091, WO 97/41102, WO 99/21832 and the like may be used.
Therefore, the disclosures of these patent publications which are incorporated herein as reference should be referred for the specific description of the compounds of the above general formulae (I) to (III).
As an amino protective radical, conventional amino protective radicals can be used and there are mentioned a lower alkanoyl radical such as acetyl, propionyl, etc.; an aroyl radical such as benzoyl, naphtoyl, etc.; an ar(lower)alkyl radical which may have a substituent such as benzyl, 4-nitrobenzyl, phenethyl, 1-phenetyl, benzhydryl, trityl, etc.; a lower alkoxycarbonyl radical such as tert-butyloxycarbonyl, etc.; an ar(lower)alkoxycarbonyl radical such as benzyloxycarbonyl, fluorenylmethoxycarbonyl, etc.

The physiologically acceptable salt of the compounds of the general formulae (I) to (IV) may be a conventional, non-toxic and physiologically acceptable salt and includes a salt with an inorganic base such as alkaline metal e.g. sodium, potassium etc., an alkaline earth metal e.g. calcium, magnesium, etc., or with ammonium; a salt with an organic base such as an organic amine e.g. triethylamine, pyridine, picoline, ethanolamine, triethanolamine, dicyclohexylamine, N,N'-dibenzylethylenediamine, etc.; an inorganic acid addition salt such as hydrochloride, hydrobromate, hydroiodate, sulfate, phosphate, etc.; an organic carboxylic or sulfonic acid addition salt such as formate, acetate, trifluoroacetate, maleate, tartrate, methanesulfonate, benzenesulfonate, p-toluenesulfonate, etc.; and a salt with a basic or acidic amino acid such as arginine, aspartate, glutamate, etc.

Among the compounds capable of binding to GPIIb/IIIa of the present invention, the compound represented by the general formula (IV) can be produced, for example, by the method described below:

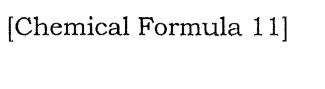

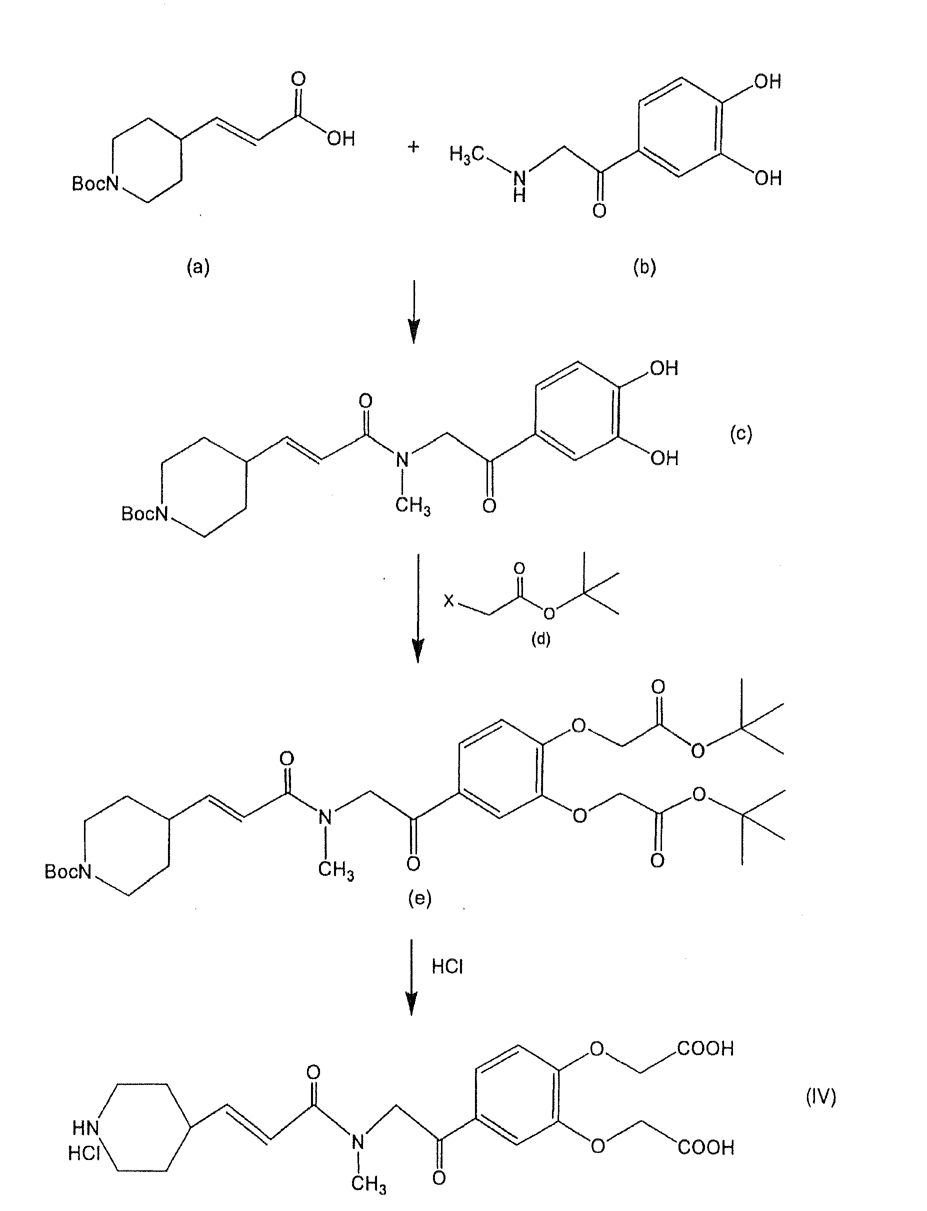

wherein X represents a halogen atom.

The reaction between the compounds of the formulae (a) and (b) is preferably carried out in the presence of a suitable condensing agent. The usable condensing agent includes 1-ethyl-3-(3-dimethylaminopropyl)carbodiimide hydrochloride, DCC (dicyclohexyl carbodiimide), etc. Among these, 1-ethyl-3-(3-dimethylaminopropyl)carbodiimide hydrochloride is preferred.

In the formula (d), a halogen atom represented by X may be a fluorine, chlorine, bromine or iodine atom and is preferably a bromine atom.
The reaction between the compound of the formulae (c) and (d) is preferably carried out in the presence of a suitable catalyst. Tetrabutylammonium iodide and the like can be used as the catalyst.
Deprotection of the compound of the formula (e) thus obtained can be carried out by a conventional method, such as by treating the compound (e) with hydrochloric acid and thus the compound (IV) can be obtained.

The compound capable of binding to GPIIb/IIIa of the present invention is the one labeled. The labeling may be the one physiologically acceptable and includes a radioactive labeling, a fluorescent labeling, a paramagnetic labeling, etc. The compound capable of binding to GPIIb/IIIa is preferably labeled with a radioactive labeling. The radioactive labeling is preferably detected by positron emission tomography and ¹¹C, ¹⁸F and the like positron emitting isotope are suitable used. The compound capable of binding to GPIIb/IIIa is preferably labeled with the positron emitting isotope.
As the method of labeling the compound capable of binding to GPIIb/IIIa, well known labeling methods can be used. For example, a method of labeling with ¹¹C can be a method of methylation using [¹¹C]CH₃I. By using such methods, the compounds represented by the formulae (I) to (IV) and physiologically acceptable salts thereof can be labeled arbitrarily.

The contrast medium for thrombus of the present invention may further comprise a conventional carrier or the other excipients which are physiologically acceptable. The physiologically acceptable carrier includes the ones normally used in the preparation of liquids, emulsions, suspensions and the like. Other agents, such as adjuvants, stabilizing agents, thickening agents, coloring agents and the like can also suitably be added.
The content of the compound capable of binding to GPIIb/IIIa in the contrast medium for thrombus of the present invention may be such an amount that the label localized at the thrombus can be detected in a detection using the contrast medium for thrombus and is selected according to the application use.

A method of detecting a thrombus which comprises the steps of administering the above contrast medium for thrombus to a mammal and detecting a label localized at the thrombus is also one of the embodiments of the present invention.
The amount of the contrast medium of the present invention to be administered is suitably selected according to the sensitivity of a detector which detects the labeled compound capable of binding to GPIIb/IIIa. The amount to be administered is preferably an amount to obtain approximately 185 to 740 MBq and approximately 185 to 740 MBq for monkeys and humans, respectively.

The step of detecting the label is carried out by, for example, positron emission tomography. Positron emission tomography includes a technique which comprises detecting a positron emitted from the labeled substance and analyzing thereof with a computer or the like to synthesize a tomographic image which reflects a specific biological properties of a tissue.

### Examples

### Production Example 1: Production of 2,2'-{[4-([methyl[(2E)-3-(4-piperidinyl)-2-propenoyl]amino}acetyl)-1,2-phenylene]bis(oxy)]diacetic acid hydrochloride

To a solution of (2E)-3-[1-(tert-butoxycarbonyl)-4-piperidinyl] acrylic acid (120 mg, 0.47 mmol) represented by the formula:

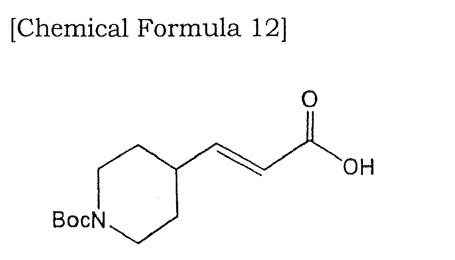

1-(3,4-dihydroxyphenyl)-2-(methylamino)ethanone (85.2 mg, 0.47 mmol) represented by the formula:

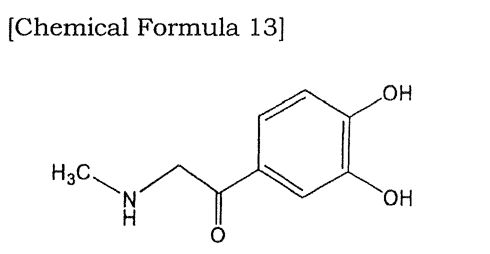

and 1-hydroxybenzotriazole (69.9 mg, 0.517 mmol) in dimethylformamide (DMF; 2 ml) was added 1-ethyl-3-(3-dimethylaminopropyl)carbodiimide hydrochloride (WSCD HCl; 99.1 mg, 0.517 mmol) with cooling on an ice bath. The reaction mixture was stirred at room temperature for 3 hours and concentrated under nitrogen flow, and the residue was partitioned between ethyl acetate and water. The mixture was extracted with ethyl acetate. The organic layer was washed with aqueous 1N hydrochloric acid solution and saturated aqueous sodium chloride solution, dried over magnesium sulfate and concentrated in vacuo. The residue was purified by preparative thin layer silica gel chromatography eluted with a mixture of chloroform-methanol (10:1) to give 4-{(1E)-3-[[2-(3,4-dihydroxyphenyl)-2-oxoethyl] (methyl)amino]-3-oxo-1-propen-1-yl}-1-piperidinecarboxylate of the formula:

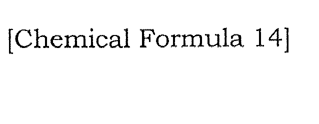

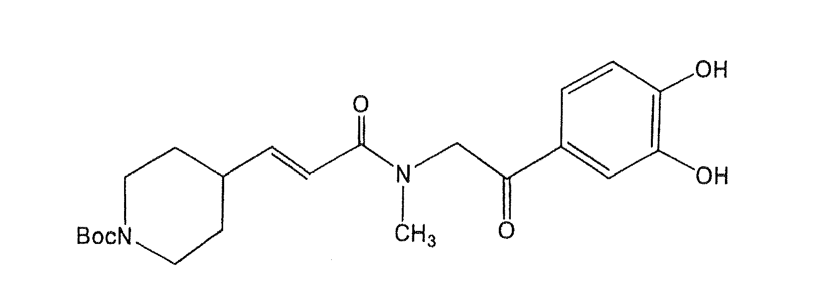

as an oil (131 mg, 66.6%).
¹H-NMR (300MHz, CDCl₃) δ; 1.33-1.45(2H, m), 1.46(9H, s), 1.70-1.82(2H, m), 2.28-2.46(1H, m), 2.71-2.84(2H, m), 2.91(3H, s), 3.49(2H, brs), 4.06-4.22 (2H, m), 4.78(2H, s), 6.37(1H, d, J=15.8Hz), 6.80(1H, d, J=8.1Hz), 6.92(1H, dd, J=15.8, 7.0Hz), 7.30(1H, d, J=8.1Hz), 7.36(1H, s); MS(ES⁺) m/z419(M+1).

To a solution of 4-{(1E)-3-[[2-(3,4-dihydroxyphenyl)-2-oxoethyl] (methyl)amino]-3-oxo-1-propen-1-yl}-1-piperidinecarboxylate (125 mg, 0.299 mmol), tert-butyl bromoacetate (122 mg, 0.627 mmol) and tetrabutylammonium iodide (11 mg, 0.03 mmol) in DMF (1.5 ml) was added potassium carbonate (86.7 mg, 0.627 mmol) with cooling on an ice bath. The reaction mixture was stirred at 60°C for 30 min and concentrated in vacuo, and the residue was partitioned between ethyl acetate and water. The mixture was extracted with ethyl acetate. The organic layer was washed with water and saturated aqueous sodium chloride solution, dried over magnesium sulfate and concentrated in vacuo. The residue was purified by preparative thin layer silica gel chromatography eluted with a mixture of chloroform-methanol (10:1) to give tert-butyl 4-{(1E)-3-[{2-[3,4-bis(2-tert-butoxy-2-oxoethoxy)phenyl]-2-oxoethyl}(methyl)amino]-3-oxo-1-propen-1-yl}-1-piperidinecarboxylate represented by the formula:

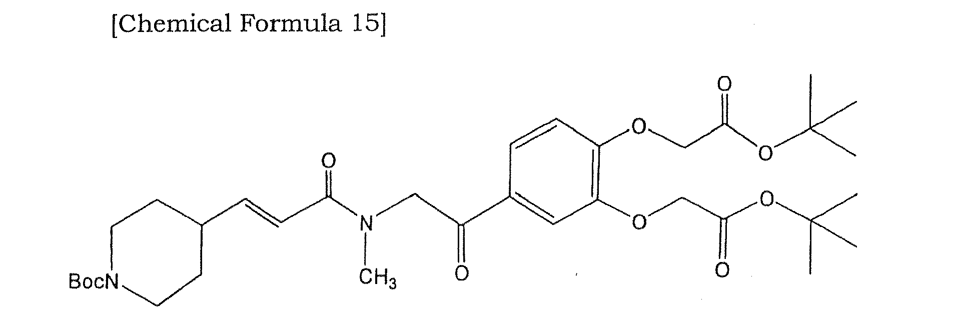

as an oil (183 mg, 94.8%).
¹H-NMR(300MHz, CDCl₃) δ; 1.24-1.51(2H,m), 1.47(9H, s), 1.48(9H, s), 1.49(9H, s), 1.71-1.80(2H, m), 2.26-2.40(1H, m), 2.66-2.84(2H, m), 3.13(3H, s), 4.03-4.20(2H, m), 4.63(2H, s), 4.68(2H, s), 4.82(2H, s), 6.33(1H, d, J=15.0Hz), 6.82(1H, d, J=8.4Hz), 6.89(1H, dd, J=15.0, 8.4Hz), 7.49(1H, s), 7.60(1H, d, J=8.4Hz); MS(ES⁺) m/z647(M+1).

To a solution of tert-butyl 4-{(1E)-3-[{2-[3,4-bis(2-tert-butoxy-2-oxoethoxy)phenyl]-2-oxoethyl}(methyl)amino]-3-oxo-1-propen-1-yl}-1-piperidinecarboxylate (5.6 mg, 8.66 µmol) in dioxane (0.5 ml) was added a solution of 4N HCl in dioxane (1.5 ml) dropwise with cooling on an ice bath. The reaction mixture was stirred at 60°C for 5 min and concentrated in vacuo to give 2,2'-{[4-([methyl[(2E)-3-(4-piperidinyl)-2-propenoyl]amino} acetyl)-1,2-phenylene]bis(oxy)]diacetic acid hydrochloride represented by the formula:

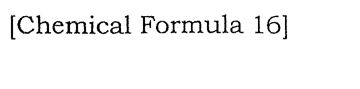

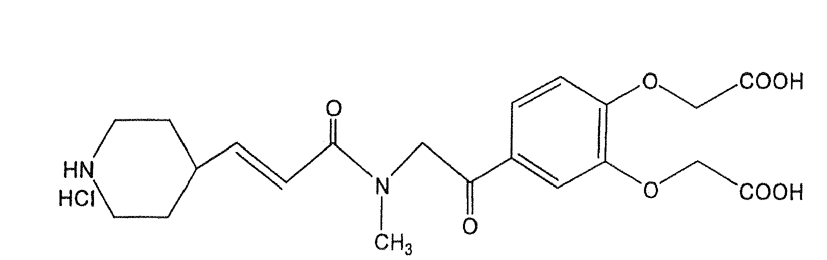

as a powder (3.8 mg, 80.3%).
¹H-NMR(300MHz, DMSO-d6) δ; 1.43-2.16(4H, m), 2.80-3.48(7H, m), 3.12(3x0.5H, s), 3.20(3x0.5H, s), 4.87(2H, brs), 4.92-4.99(4H, m), 6.29(1H, brd), 6.57-6.79(1H, m), 7.07-7.19(1H, m), 7.50(1H, brs), 7.72-7.80(1H, m), 8.86-9.13(1H, brs); MS(ES⁺) m/z435(M+1).

### Production Example 2: Production of N-[(3R)-1-[3-(4-piperidyl)propionyl]-3-piperidylcarbonyl]-2(S)-methoxyc arbonylamino-β-alanine

The compound of the above formula (III-1) was produced according to the method described in WO 01/60813 (Example 19).

### Production Example 3

A compound represented by the formula: 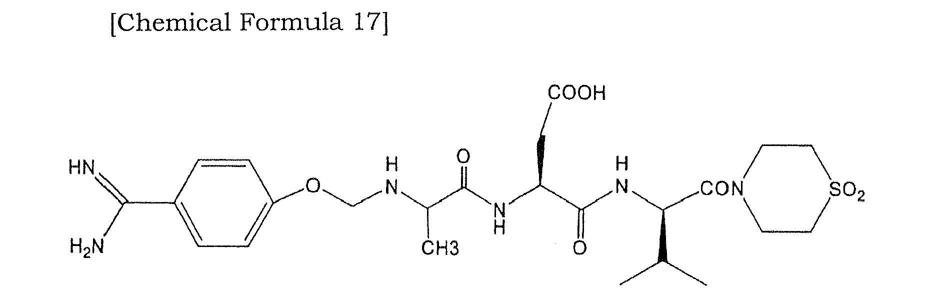 was produced according to the well known method.

### Production Example 4

A compound represented by the formula:

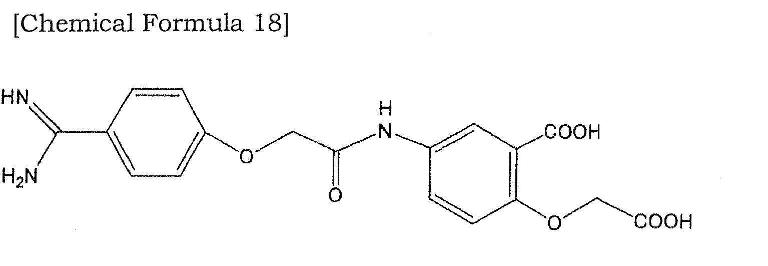

was produced according to the well known method.

### Production Example 5

A compound represented by the formula: 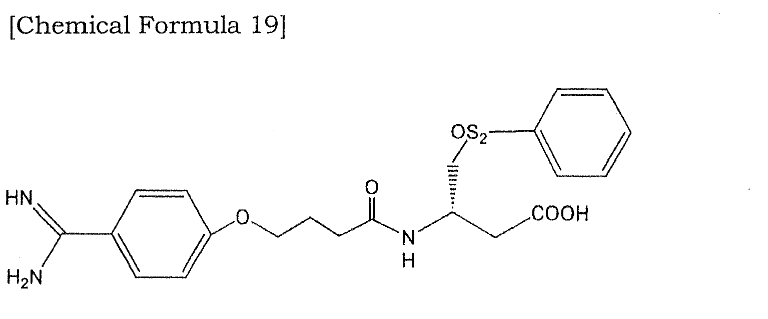 was produced according to the well known method.

### Production Example 6

A compound represented by the formula:

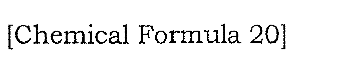

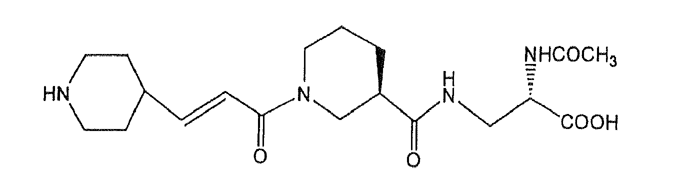

was produced according to the method described in WO 01/60813.

### Production Example 7

A compound represented by the formula:

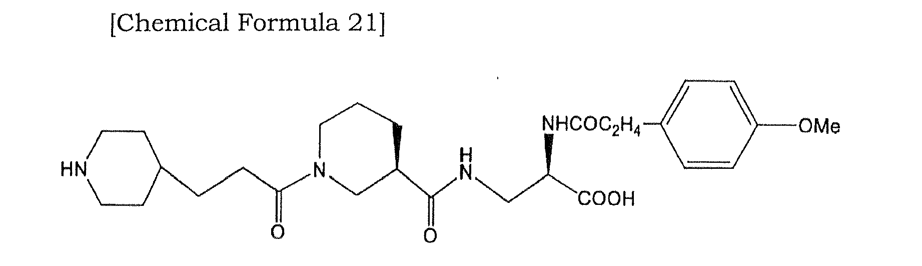

was produced according to the method described in WO 01/60813.

### Production Example 8

A compound represented by the formula:

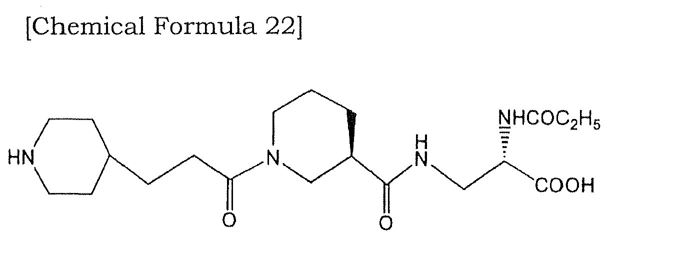

was produced according to the method described in WO 01/60813.

### Production Example 9

A compound represented by the formula:

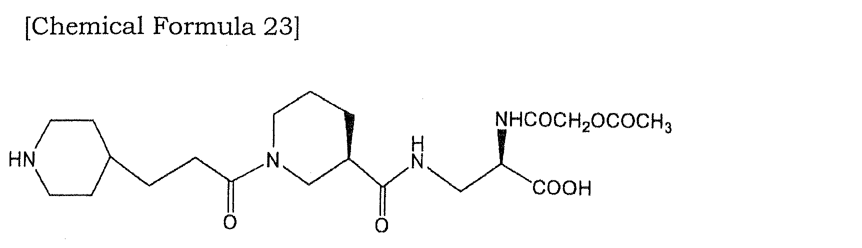

was produced according to the method described in WO 01/60813.

### Production Example 10

A compound represented by the formula:

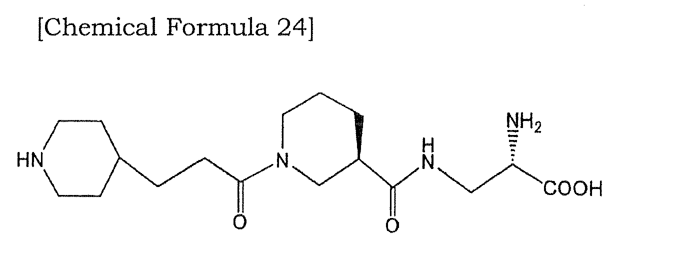

was produced according to the method described in WO 01/60813.

### Production Example 11

A compound represented by the formula:

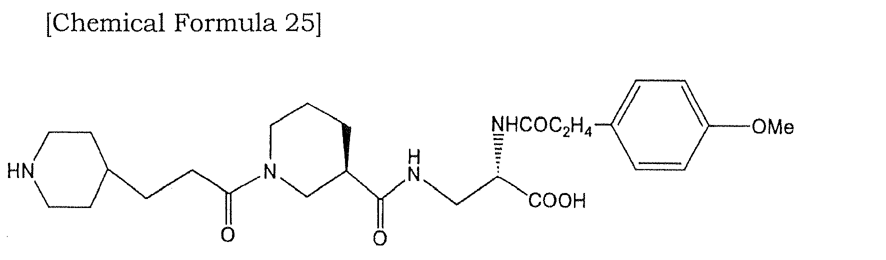

was produced according to the method described in WO 01/60813.

### Production Example 12

A compound represented by the formula:

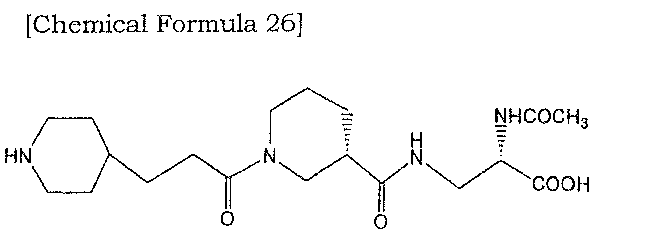

was produced according to the method described in WO 01/60813.

### Production Example 13

A compound represented by the formula:

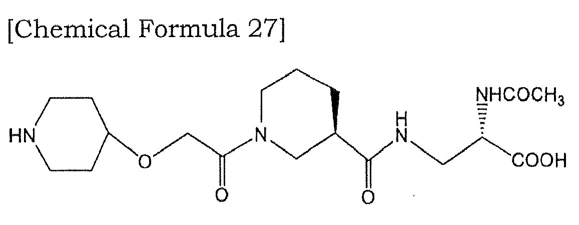

was produced according to the method described in WO 01/60813.

### Production Example 14

A compound represented by the formula:

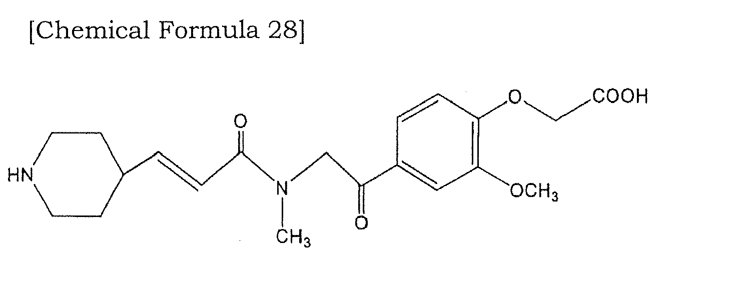

was produced according to the well known method.

### Production Example 15

A compound represented by the formula:

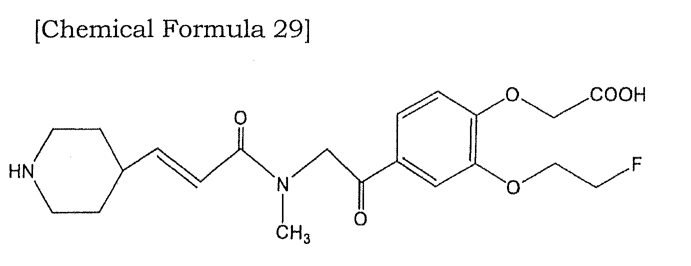

was produced according to the well known method.

### Production Example 16

A compound represented by the formula:

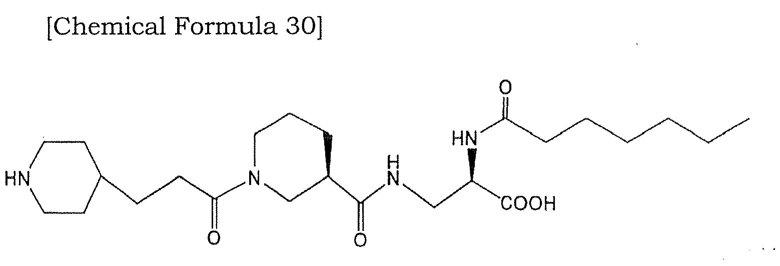

was produced according to the method described in WO 01/60813.

### Production Example 17

A compound represented by the formula:

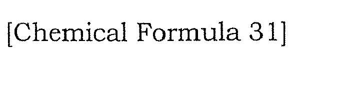

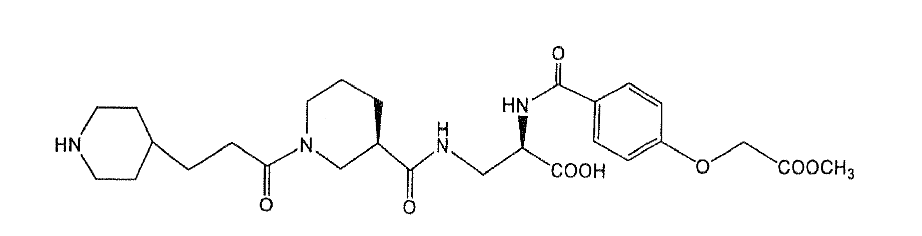

was produced according to the method described in WO 01/60813.

### Production Example 18

A compound represented by the formula:

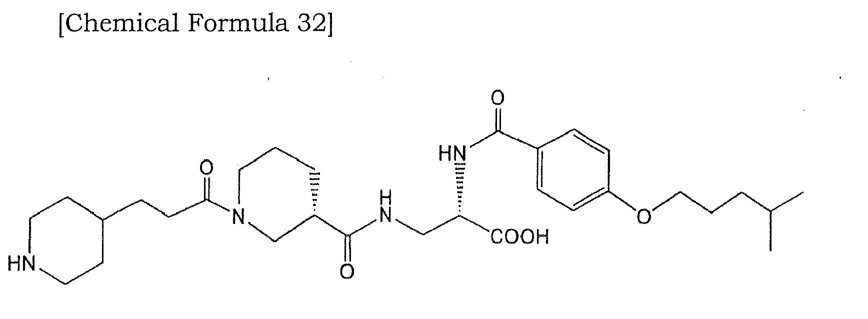

was produced according to the method described in WO 01/60813.

### Production Example 19

A compound represented by the formula:

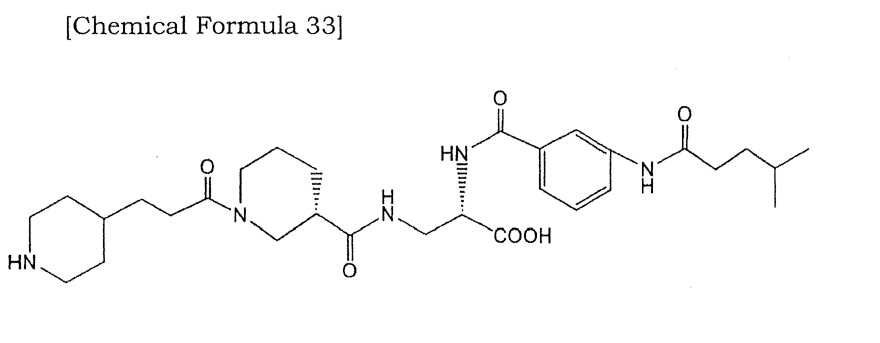

was produced according to the method described in WO 01/60813.

### Production Example 20

A compound represented by the formula:

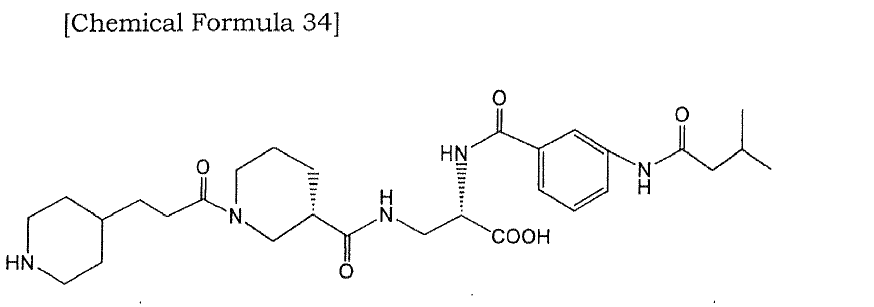

was produced according to the method described in WO 01/60813.

### Examples 1 to 20 and Reference Examples 1 to 4

The following tests were carried out with compounds prepared in Production Examples 1 to 20. As Reference Examples 1 to 4, Echistatin, which is a venom protein known as binding substance to GPIIb/IIIa, Tirofiban (MK383), Lamifiban (Ro44-9883) and FK633, which are antithrombotic drugs, were used to carry out the following tests as well.
Results are shown in Tables 1-1 to 1-3.

### Test 1: Measurement of inhibitory activity of adenosine diphosphate (ADP)-induced platelet aggregation

Platelet rich plasma (PRP) containing 3 x 10⁸ platelets/ml was prepared from human blood. To 225 µl of PRP, 25 µl of a solution of the test compound was added and stirred at 37°C for 2 min. To the solution, 5 µl of ADP (final concentration: 2.5 µM) was added as an aggregation inducing agent. Aggregation was measured with aggregometer (NBS HEMA-TRACER 801). The procedure was as follows. Light transmittance of PRP was calibrated to 100%. PRP was incubated in the aggregometer at 37°C for 2 min. ADP was added when full response of the platelet aggregation was obtained, and the change in light transmittance was monitored by PL500 recorder (Yokogawa, Japan). Percent inhibition of aggregation by the test compound was calculated by comparison with the aggregation in the absence of the test compound. The activity of an inducing substance (test compound) is represented as a value of IC₅₀, i.e., a dose required for the complete inhibition of the platelet aggregation.
It shows that the smaller the value from Test 1 is, the higher the binding ability of the test compound toward the active GPIIb/IIIa is.

### Test 2: Measurement of suppression activity of fibrinogen adhesion of prostaglandin E1 (PGE1)-treated platelet

Venous blood was collected onto sodium citrate. Platelet rich plasma (PRP) was prepared by centrifugation of whole blood. Platelets were washed with modified HEPES-Tyrode's buffer (129 mM NaCl, 2.8 mM KCl, 0.8 mM KH₂PO₄, 8.9 mM NaHCO₃, 0.8 mM MgCl₂, 10 mM HEPES, 5.5 mM Glucose, 0.1% BSA, pH 7.4) containing 1µM PGE1. After washing, platelets were suspended in modified HEPES-Tyrode's buffer containing 1.0 mM CaCl₂ and 1 µM PGE1 and the number of platelet was adjusted.

Adhesion assay was carried out as follows. 96-well microtiter plates were coated with 1 µg/well of human fibrinogen. The plates were then blocked with 1% BSA. After washing the plates with the buffer, the washed platelets were added to each well in the presence of the test compound or buffer and incubated at 37°C for 30 min. The plates were then washed three times with buffer. The number of adhered cells was determined by measuring the acid phosphatase activity of cells using a microplate reader at 410 nm. Percent inhibition of adhesion in the sample treated with the test compound was calculated by comparison with the adhesion in absence of the test compound. The activity of the test compound was represented by a value of IC₅₀, i.e. a dose required for the complete inhibition of the platelet adhesion.
It shows that the bigger the value from Test 2 is, the lower the binding ability of the test compound toward the resting GPIIb/IIIa is.

The IC₅₀ value (A) obtained in Test 1 and the IC₅₀ value (R) obtained in Test 2 were used to calculate the R/A ratio, and selective binding ability of the test compounds toward the active GPIIb/IIIa was evaluated.
Results are shown in Tables 1-1 to 1-4.

**[Table 1]**

| | Compound Structure | A Inhibition of platelet aggregation IC₅₀ (nM) | R Suppression of fibrinogen adhesion IC₅₀ (nM) | R/A ratio |
|---|---|---|---|---|
| Ex. 1 | 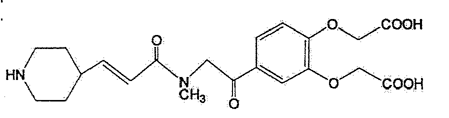 | 135 | 7900 | 59 |
| Ex. 2 | 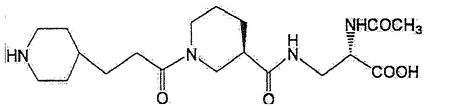 | 53 | 298 | 5. 6 |
| Ex. 3 | 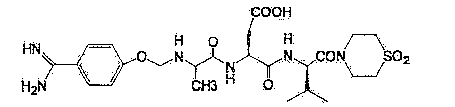 | 73 | 1350 | 18 |
| Ex. 4 | 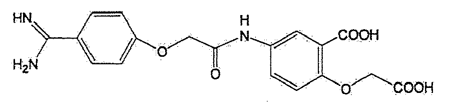 | 260 | 1630 | 6. 3 |
| Ex. 5 | 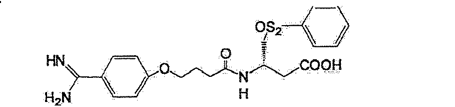 | 80 | 770 | 9.6 |
| Ex. 6 | 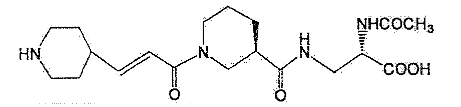 | 56 | 434 | 7.8 |
| Ex. 7 | 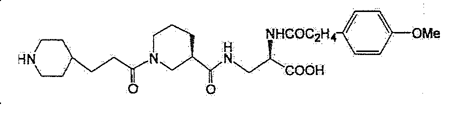 | 35 | 533 | 15 |

**[Table 1-2]**

| | Compound Structure | A Inhibition of platelet aggregation IC₅₀ (nM) | R Suppression of fibrinogen adhesion IC₅₀ (nM) | R/A ratio |
|---|---|---|---|---|
| Ex. 8 | 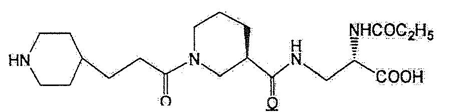 | 49 | 1525 | 11 |
| Ex. 9 | 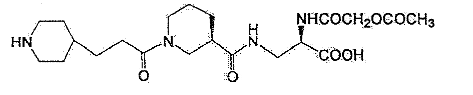 | 55 | 727 | 13 |
| Ex. 10 | 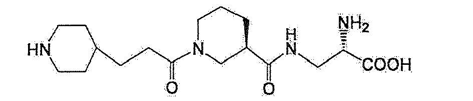 | 272 | 2510 | 9.2 |
| Ex. 11 | 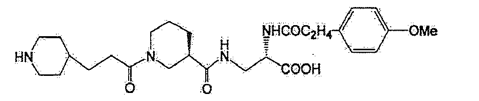 | 88 | 479 | 6. 5 |
| Ex. 12 | 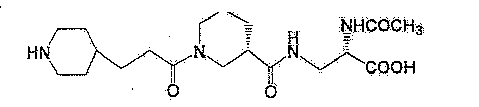 | 791 | 5160 | 6.5 |
| Ex. 13 | 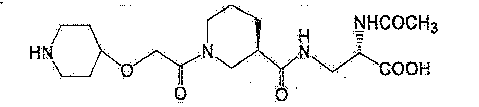 | 817 | 10600 | 13 |
| Ex. 14 | 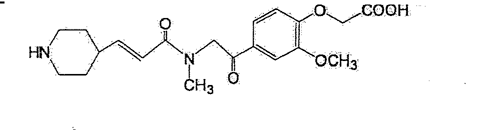 | 291 | 4050 | 14 |

**[Table 1-3]**

| | Compound Structure | A Inhibition of platelet aggregation IC₅₀ (nM) | R Suppression of fibrinogen adhesion IC₅₀ (nM) | R/A ratio |
|---|---|---|---|---|
| Ex. 15 | 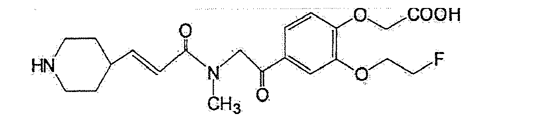 | 320 | 6210 | 19 |
| Ex. 16 | 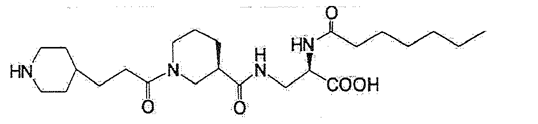 | 32. 4 | 198 | 6.1 |
| Ex. 17 | 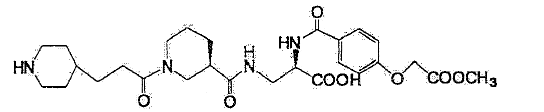 | 29.9 | 486 | 16 |
| Ex. 18 | 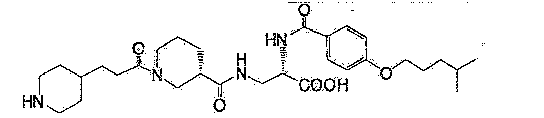 | 6.1 | 48 | 7. 9 |
| Ex. 19 | 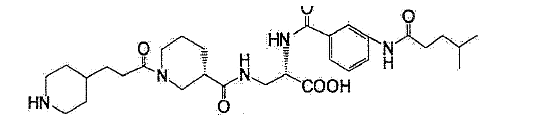 | 9. 1 | 151 | 17 |
| Ex. 20 | 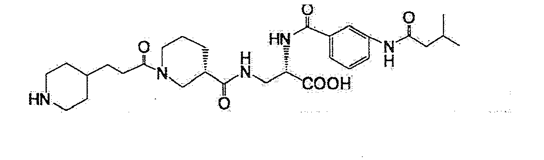 | 30 | 239 | 8.0 |

**[Table 1-4]**

| | Compound Structure | A Inhibition of platelet aggregation IC₅₀ (nM) | R Suppression of fibrinogen adhesion IC₅₀ (nM) | R/A ratio |
|---|---|---|---|---|
| Reference Ex. 1 | | 0.36µg/ml | 0.14µg/ml | 0.38 |
| Reference Ex. 2 | 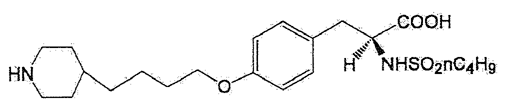 | 46 | 36 | 0. 78 |
| Reference Ex. 3 | 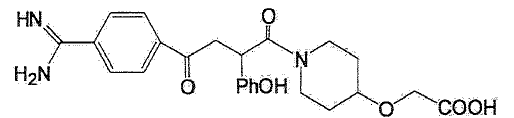 | 45 | 80 | 1.8 |
| Reference Ex. 4 | 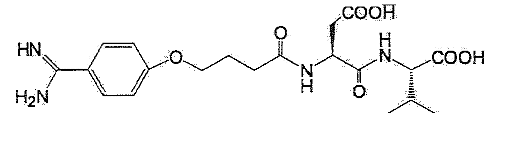 | 103 | 1320 | 13 |

As obvious from Tables 1-1 to 1-4, the compounds capable of binding to GPIIb/IIIa for the present contrast medium for thrombus have high R/A values. Therefore, it is shown that it has high selective binding ability toward the active GPIIb/IIIa.
The compounds capable of binding to GPIIb/IIIa used for the present contrast medium for thrombus show lower IC₅₀ values in the inhibitory test of ADP-induced platelet aggregation than ICso values disclosed in the prior arts. Therefore, it is obvious that these compounds capable of binding to GPIIb/IIIa bind to the GPIIb/IIIa on the surface of the platelets activated by ADP.

### Production Example 21: Production of the contrast medium for thrombus

To a solution of (2E)-3-[1-(tert-butoxycarbonyl)-4-piperidinyl] acrylic acid (1.6 g, 6.27 mmol), di-tert-butyl 2,2'-[[4-(aminoacetyl)-1,2-phenylene]bis(oxy)]diacetate hydrochloride (2.71 g, 6.27 mmol) represented by the formula:

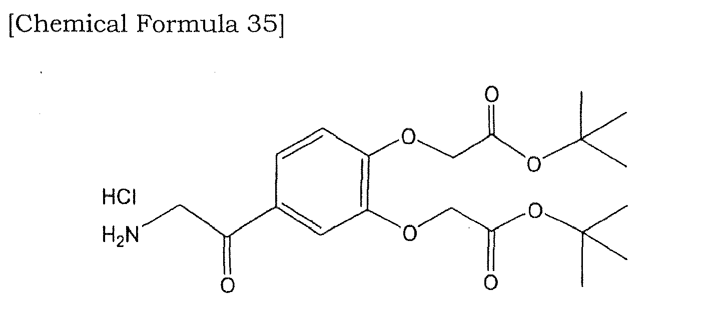

and bromotripyrrolidionophosphonium hexafluorophosphate (3.42 g, 6.58 mmol) in DMF (10 ml) was added N,N-diisopropylethylamine (2.51 g, 19.1 mmol) dropwise with cooling on an ice bath. The reaction mixture was stirred at 0°C for 20 min and then at room temperature for 30 min. The mixture was concentrated in vacuo, and the residue was partitioned between ethyl acetate and water. The mixture was extracted with ethyl acetate. The organic layer was washed with 0.5N aqueous potassium hydrogen sulfate solution, saturated aqueous sodium bicarbonate solution and saturated aqueous sodium chloride solution, dried over magnesium sulfate, and concentrated in vacuo. The residue was purified by silica gel column chromatography eluted with a mixture of chloroform-ethyl acetate (9: 1) to give tert-butyl 4-[(1E)-3-({2-[3,4-bis(2-tert-butoxy-2-oxoethoxy) phenyl]-2-oxoethyl}amino)-3-oxo-1-propen-1-yl]-1-piperidinecarboxylate represented by the formula:

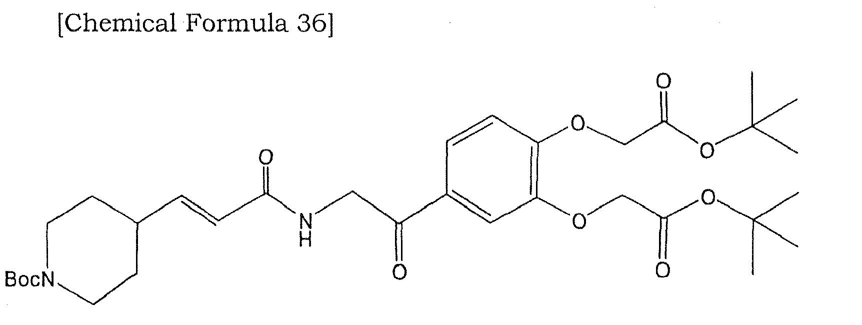

as an amorphous.
¹H-NMR(300MHz, DMSO-d6) δ; 1.18-1.36(2H, m), 1.49(9H, s), 1.53(18H, s), 1.71-1.84(2H, m), 2.32-2.47(1H, m), 2.74-2.99(2H, m), 3.94-4.11(2H, m), 4.71(2H, d, J=5.5Hz), 4.85(2H, s), 4.91(2H, s), 6.14(1H, d, J=15.4Hz), 6.70(1H, dd, J=15.4, 6.2Hz), 7.09(1H, d, J=8.8Hz), 7.49(1H, d, J=2.2Hz), 7.76(1H, dd, J=8.8, 2.2Hz), 8.36(1H, brt, J=5.5Hz); MS(ES⁺)m/z was not detected.

To a solution of tert-butyl 4-[(1E)-3-({2-[3,4-bis(2-tert-butoxy-2-oxoethoxy) phenyl]-2-oxoethyl}amino)-3-oxo-1-propen-1-yl]-1-piperidi necarboxylate (3.6 g, 5.69 mmol) and tert-butyldimethylsilyl chloride (1.29 g, 8.53 mmol) in CH₂Cl₂ (10 ml) was added 1,8-diazabicyclo[5.4.0]undec-7-ene (1.47 g, 9.67 mmol) dropwise with cooling on an ice bath. The reaction mixture was stirred at room temperature for 2 hours and concentrated in vacuo, and the residue was partitioned between ethyl acetate and water. The mixture was extracted with ethyl acetate. The organic layer was washed with 1N aqueous hydrogen chloride solution, saturated aqueous sodium bicarbonate solution and saturated aqueous sodium chloride solution, dried over magnesium sulfate, and concentrated in vacuo. The residue was purified by silica gel column chromatography eluted with a mixture of hexane-ethyl acetate (4:1) to give 4-{(1E)-3-[((Z)-2-[3,4-bis(2-tert-butoxy-2-oxoethoxy)phenyl]-2-{[tert-butyl(dimethyl)silyl]oxy}vinyl)amino]-3-oxo-1-propen-1-yl}-1-piperidinecarboxylate represented by the formula:

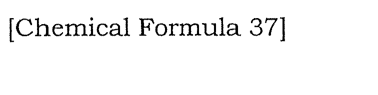

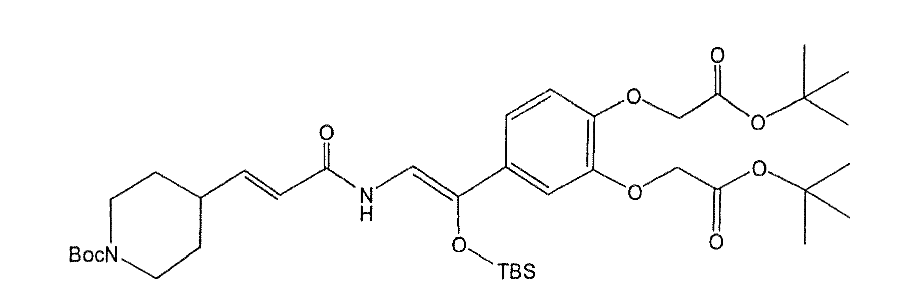

as an amorphous.
¹H-NMR(300MHz, DMSO-d6) δ; 0.02-0.08(6H, m), 0.98(9H, s), 1. 15-1.30(2H, m), 1.43(9H, s), 1.44(9H, s), 1.46(9H, s), 1.61-1.78(2H, m), 2.27-2.48(1H, m), 2.68-2.91(2H, m), 3.88-4.04(2H, m), 4.70(2H, s), 4.72(2H, s), 6.23(1H, d, J=15.8Hz), 6.71(1H, d, J=6.2Hz), 6.76(1H, d, J=6.2Hz), 6.85-6.94(2H, m), 7.01(1H, dd, J=8.1, 1.8Hz); MS(ES⁺)m/z was not detected.

To a solution of 4-{(1E)-3-[((Z)-2-[3,4-bis(2-tert-butoxy-2-oxoethoxy)phenyl]-2-{[tert-butyl(dimethyl)silyl]oxy}vinyl)amino]-3-oxo-1-propen-1-yl}-1- piperidinecarboxylate in DMF (2 ml) was added sodium hydride (17.7 mg, 736 mmol) portionwise with cooling on an ice bath and stirred at 0°C for 5 min. Then [¹¹C]CH₃I (105 mg, 736 mmol) was added to the reaction mixture at the same temperature, and the mixture was stirred for 5 min and stirred at room temperature for 5 min. The mixture was partitioned between ethyl acetate and phosphate buffer standard solution (pH 6.86). The mixture was extracted with ethyl acetate. The organic layer was washed with water and saturated aqueous sodium chloride solution, dried over magnesium sulfate, and concentrated in vacuo. The residue was purified by silica gel column chromatography eluted with a mixture of hexane-ethyl acetate (1:1) to give 4{(1E)-3-[((Z)-2-[3,4-bis(2-tert-butoxy-2-oxoethoxy)phenyl]-2-{[tert-butyl(dimethyl)silyl]oxy}vinyl) ([¹¹C]methyl)amino]-3-oxo-1-propen-1-yl}-1-piperidinecarboxylate represented by the formula:

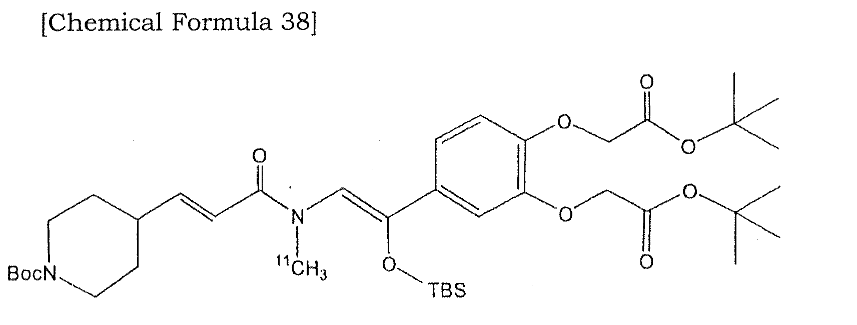

as an amorphous.

To a mixture of trifluoroacetic acid (TFA; 1 ml) and CH₂Cl₂ (1 ml) was added 4{(1E)-3-[((Z)-2-[3,4-bis(2-tert-butoxy-2-oxoethoxy) phenyl]-2-{[tert-butyl(dimethyl)silyl]oxy}vinyl) ([¹¹C]methyl)amino]-3-oxo-1-propen-1-yl}-1-piperidinecarboxylate in CH₂Cl₂ (0.5 ml) dropwise with cooling on an ice bath. The reaction mixture was stirred at room temperature for 30 min. The mixture was concentrated in vacuo and the residue was purified by ODS chromatography eluted with a mixture of acetonitrile-water (10:1) to give 2,2'-[[4-({[¹¹C]methyl[(2E)-3-(4-piperidinyl)-2-propenoyl]amino}acetyl)-1,2-phenylene]bis(oxy)]diacetic acid trifluoroacetate represented by the formula:

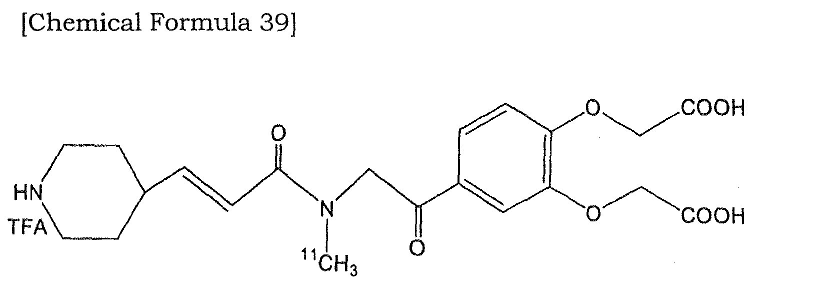

as an amorphous.

### Production Example 22: Production of the contrast medium for thrombus

A solution of the compound prepared in Production Example 15 (13.0 mg, 30 µmol) in acetonitrile (1.5 ml) was added to a solution of [¹⁸F]⁻ ion. The mixture was heated at 85°C for 20 min. After the mixture was cooled to room temperature, 4M HCl (1.0 ml) was added. The mixture was heated at 100°C for 10 min. Thus obtained mixture was loaded on an HPLC column (YMC-Pack C18 Pro, 10 x 250 mm, YMC Co., Ltd, Japan) eluted with 0.1% acetonitrile-0.05M NH₄OAc to give the contrast medium for thrombus labeled with [¹⁸F].

### Examples 21 and 22

Kinetics of the labeled compounds in the body of monkey (*Macaca fascicularis*) was studied by using the contrast mediums labeled with [¹¹C] and [¹⁸F] prepared in Production Examples 21 and 22, respectively.
According to the canine saphenous vein (which is formed with a confluence of dorsal digital veins of hallux and arcuate veins of foot, and ascends in front of a medial malleolus and behind of a medial condyle of femur, crosses a saphenous opening of patellar retinaculum and pour into a femoral vein over a femoral triangle) thrombosis model (Knight L.C. et al., Thromb Haemost., 1998, 80, p.845-851 and Lister-James L. et al., J. Nucl Med. 1996, 37, p.775-781), a platinum embolization coil (Boston Scientific Japan) was inserted and placed in the right femoral vein of the monkey while anesthetized and wound was sutured.

Three hours after the insertion of the coil, the contrast mediums prepared in Production Examples 21 and 22, respectively, were administered intravenously (740 MBq/2 ml-saline). Imaging was performed for 90 min with a high-resolution positron emission tomography (PET) scanner (SHR-7700, Hamamatsu Photonics K.K., Japan). After PET measurement, the coil was removed, and the right femoral vein was collected to obtain a thrombus sample. Thigh muscle was collected, and saphenous artery was punctured to collect an arterial blood sample. The thrombus, thigh muscle and arterial blood samples were weighed and their radioactivity was measured with gamma counter (1480 WIZARD, Wallac Oy, Finland).
Results are shown in the Table 2 below. Data was calculated as percent of injected dose (%ID/kg/g).

**[Table 2]**

| Accumulation of radioactivity at thrombus, blood and muscle at 90 min after the administration of the contrast medium in monkey thrombus model | | | | | |
|---|---|---|---|---|---|
| Contrast medium | Thrombus (%ID/g/kg) | Blood (%ID/g/kg) | Muscle (%ID/g/kg) | Thrombus/Blood ratio | Thrombus/Muscle ratio |
| Production Ex. 21 ([¹¹C]label) | 1.902±1.132 | 0.084±0.012 | 0.021±0.005 | 24.2±17.9 | 94.8±65.0 |
| Production Ex. 22 ([¹⁸F]label) | 0.208±0.028 | 0.043±0.004 | 0.013±0.002 | 4.8±0.8 | 16.3±2.7 |

As observed from Table 2, the contrast mediums of Production Example 21 and 22 were accumulated in the thrombus with the ratio of approximately 24-fold and 4.8-fold (relative to blood) as well as approximately 95-fold and 16-fold (relative to muscle), respectively. Therefore, the contrast medium for thrombus of the present invention is demonstrated to specifically bind to the thrombus.
Moreover, it was possible to carry out the PET imaging of thrombus with low background noise and high resolution.

## Claims

1. A contrast medium for thrombus which comprises, as an active substance, a substance obtained by labeling a compound capable of binding to glycoprotein IIb/IIIa.

2. A contrast medium for thrombus which comprises, as an active substance, a substance obtained by labeling a compound capable of binding to glycoprotein IIb/IIIa selected from compounds represented by the general formula (I): 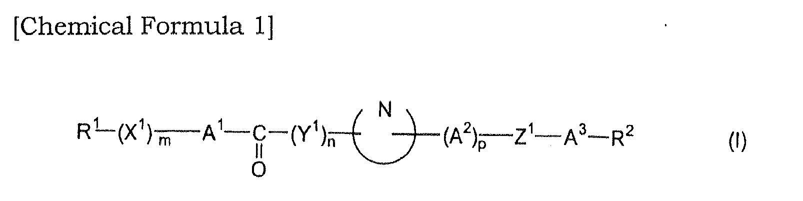 wherein
R¹ represents an N-containing cycloalkyl radical which may have one or more substituents; 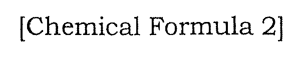 R² represents a carboxy or protected carboxy radical;
A¹ represents a lower alkylene, lower alkanyl-ylidene or lower alkenylene radical, each of which may have one or more substituents;
A² represents a lower alkylene radical;
A³ represents a lower alkylene radical which may have one or more substituents;
a moiety represented by 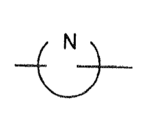 is a N-containing heterocyclic radical represented by the formula: 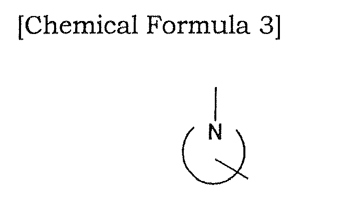 which may have one or more substituents;
X¹ represents O, S or NH;
Y¹ represents NH; and
Z¹ represents 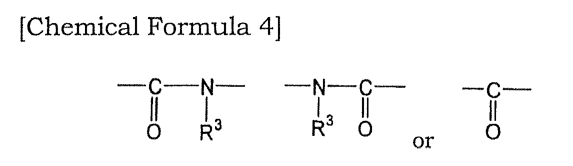 wherein R³ represents a hydrogen atom or a lower alkyl radical; and
m, n and p are the same or different and represent an integer of 0 or 1, respectively;
and a physiologically acceptable salt thereof,
compounds represented by the general formula (II): 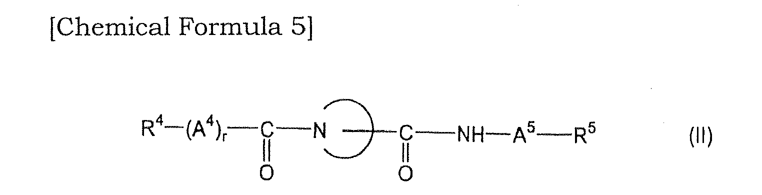 wherein
R⁴ represents a piperidyl, tetrahydropyridyl, azetidinyl or tetrahydroisoquinolyl radical and these piperidyl, tetrahydropyridyl, azetidinyl and tetrahydroisoquinolyl radicals may have an amino protective group;
R⁵ represents a carboxy or protected carboxy radical;
A⁴ represents a lower alkylene, lower alkanyl-ylidene, lower alkenylene, cyclo(lower)alkylene or arylene radical;
A⁵ represents a lower alkylene radical which may have one or more substituents or an arylene radical;
a moiety represented by 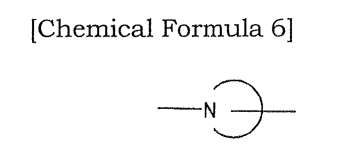 represents a piperidinediyl or tetrahydroisoquinolinediyl radical; and
r represents an integer of 0 or 1;
and a physiologically acceptable salt thereof,
compounds represented by the general formula (III): 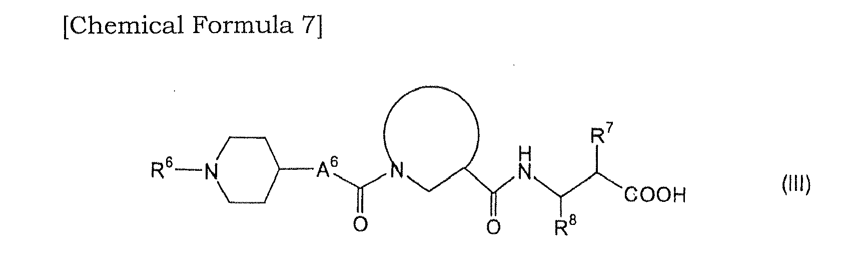 wherein
R⁶ represents a hydrogen atom or an amino protective group;
A⁶ represents a lower alkylene or lower alkenylene radical;
R⁷ represents a hydrogen atom; a lower alkanoyl radical which may be substituted with amino, lower alkanoylamino, ar(lower)alkoxycarbonylamino, aryl, aroylamino, carboxy, lower alkoxycarbonylamino, ar(lower)alkoxy, lower alkoxycarbonyl, lower alkanoyloxy, lower alkoxy or hydroxyl, among which aryl and aroylamino may further be substituted with carboxy, lower alkoxy or lower alkoxycarbonyl; a lower alkoxycarbonyl radical which may be substituted with lower alkoxy, aryl or cyclo(lower)alkyl; a lower alkenyloxycarbonyl radical; a di(lower)alkylaminosulphonyl radical; a cycloalkanoyl radical which may be substituted with lower alkoxy; an aroyl radical which may be substituted with (C₃-C₆) alkoxy, carbamoyl(lower)alkoxy, N-(lower)alkylcarbamoyl(lower)alkoxy, N,N-di(lower)alkylcarbamoyl(lower)alkoxy, lower alkoxycarbonyl, nitro, cyano, carboxy, carboxy(lower)alkoxy, ar(lower)alkoxy, lower alkoxycarbonyl(lower)alkoxy, cyclo(lower)alkoxy, lower alkoxycarbonylamino, cyclo(lower)alkyl(lower)alkoxy, lower alkanoylamino or lower alkylcarbamoyl; an aryloxycarbonyl radical; a heterocyclylcarbonyl radical; an amino radical which may be substituted with an acyl radical selected from the group consisting of a protected carboxycarbonyl radical and a heterocyclyloxycarbonyl radical;
R⁸ represents a hydrogen atom or an aryl or aralkyl radical which may be substituted with one or more hydroxyl and/or lower alkoxy;
a moiety represented by the formula: 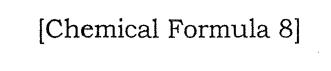 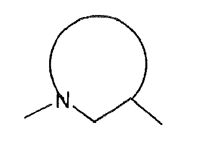 represents a divalent N-containing, 6 to 8-membered heterocyclic radical;
and a physiologically acceptable salt thereof, and
compounds represented by the formula (IV): 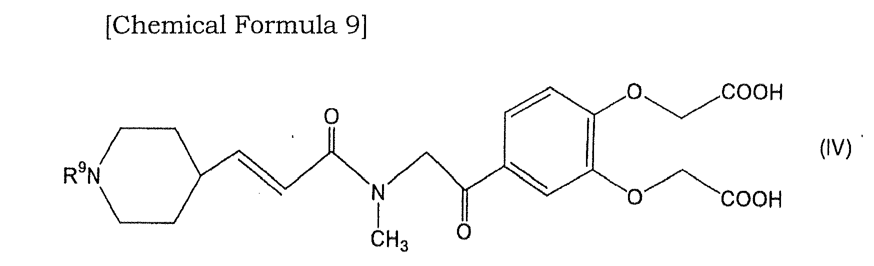 wherein R⁹ represents a hydrogen atom or an amino protective group; and a physiologically acceptable salt thereof.

3. The contrast medium for thrombus according to claim 2, wherein the compound capable of binding to glycoprotein IIb/IIIa is a compound represented by the formula (III-1): 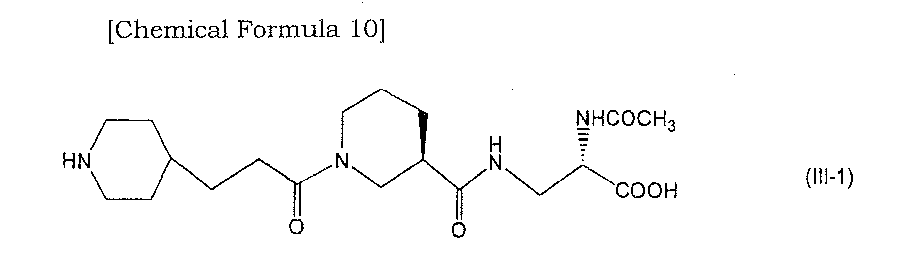 or a physiologically acceptable salt thereof.

4. The contrast medium for thrombus according to any one of claims 1 to 3, wherein the compound capable of binding to glycoprotein IIb/IIIa is labeled with a positron emitting isotope.

5. The contrast medium for thrombus according to any one of claims 1 to 4, wherein the compound capable of binding to glycoprotein IIb/IIIa is labeled with ¹¹C.

6. A compound represented by the general formula (IV): 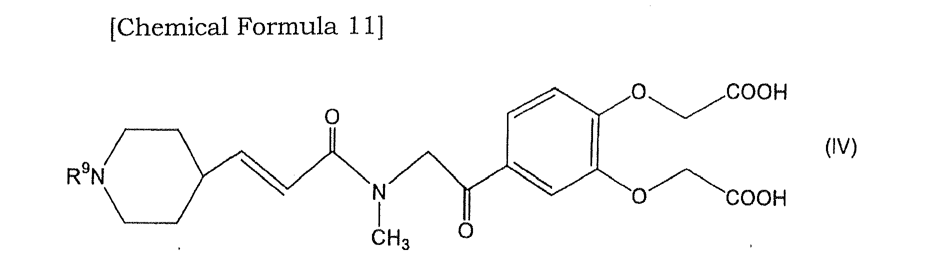 wherein R⁹ represents a hydrogen atom or an amino protective group, and a physiologically acceptable salt thereof.

7. A method of detecting a thrombus which comprises the steps of administering the contrast medium for thrombus according to any one of claims 1 to 5 to a mammal and detecting a label localized to the thrombus.

8. The method according to claim 7, wherein the detection step is carried out by positron emission tomography.
